# DEMANDE DE BREVET EUROPEEN

(11) **EP 1 992 330 A2**
(43) Date de publication de la demande: **19.11.2008**
(21) Numéro de dépôt: 08155811.6
(22) Date de dépôt: 07.05.2008
(51) Int. Cl.: A61K 8/49, A61Q 5/10

(54) **Procédé de coloration des fibres kératiniques comprenant l'application d'une base d'oxydation aminopyrazolopyridine en l'absence d'agent oxydant**

(30) Priorité: 09.05.2007 FR 0754948
(71) Demandeur: L'Oreal, 75008 Paris (FR)
(72) Inventeur: Saunier, Jean-Baptiste, 75014, PARIS (FR); Fadli, Aziz, 77500, CHELLES (FR)
(74) Mandataire: Fevrier, Murielle Françoise E.

(57) **Abrégé**

L'invention a pour objet un procédé de coloration des fibres kératiniques à partir d'une base aminopyrazolopyridine, le procédé de coloration étant mis en oeuvre en l'absence d'agent oxydant chimique.

La présente invention permet en particulier d'obtenir une coloration des fibres kératiniques, tenace, résistante à la lumière et au lavage sans dégradation des fibres kératiniques.

La composition de la présente invention permet en particulier d'obtenir une coloration des fibres kératiniques aux nuances variées, puissantes, esthétiques, peu sélectives et résistant bien aux diverses agressions que peuvent subir les cheveux sans dégradation des fibres kératiniques

## Description

L'invention a pour objet un procédé de coloration des fibres kératiniques à partir d'une base aminopyrazolopyridine.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est déjà connu d'utiliser des bases d'oxydation aminopyrazolopyridine pour la teinture des fibres kératiniques en présence d'un agent oxydant tel que le peroxyde d'hydrogène, notamment dans la demande de brevet FR 2 801 308. Ces bases permettent d'obtenir des nuances variées.

Le but de la présente invention est d'obtenir un procédé pour la coloration des cheveux qui présente des propriétés tinctoriales améliorées en terme de puissance et de résistance aux agents extérieurs.

Ce but est atteint avec la présente invention qui a pour objet un procédé de coloration des fibres kératiniques comprenant, l'application sur les fibres kératiniques d'une composition comprenant dans un milieu de teinture approprié,
- une ou plusieurs bases d'oxydation aminopyrazolopyridine choisie parmi les bases de formule (I) ou (II), 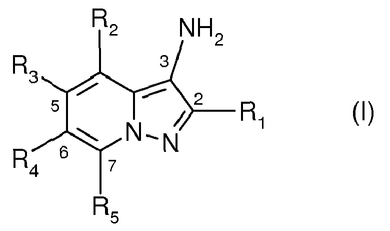 dans laquelle :
   - R₁, R₂, R₃, R₄ et R₅, identiques ou différents, représentent un atome d'hydrogène ou d'halogène ; un radical -NHSO₃H ; un radical hydroxyle ; un radical (C₁-C₄)alkyle ; un radical (C₁-C₄)alkoxy ; un radical (C₁-C₄)alkylthio ; mono(C₁-C₄)alkylamino ; un radical di(C₁-C₄)alkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre ; un hétérocycle ; un radical nitro ; un radical phényle ; un radical carbonyle ; un radical alkoxy(C,-C₄)carbonyle ; un radical carboxamido ; un radical cyano ; un radical amino ; un radical sulfonyle; un radical -CO₂H, un radical -SO₃H ; un radical -PO₃H₂ ; un radical -PO₄H₂; ou un groupement : 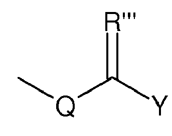 dans lequel R"' représente un atome d'oxygène ou d'azote, Q représente un atome d'oxygène, un groupement NH ou NH(C₁-C₄)alkyle, et Y représente un radical hydroxyle, amino, alkyle en C₁-C₄, (C₁-C₄)alkoxy, (C₁-C₄)alkylamino, ou di(C₁-C₄)alkylamino. 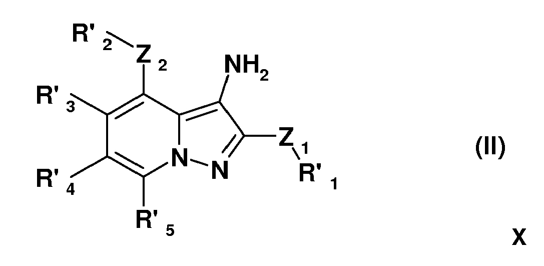 dans laquelle
      - Z₁ et Z₂ représentent indépendamment
         - une liaison covalente simple,
         - un radical divalent choisi parmi
         - un radical -O(CH₂)ₚ-, p désignant un nombre entier allant de 0 à 6
         - un radical -NR'₆(CH₂)_{q}(C₆H₄)ₜ-, q désignant un nombre entier allant de 0 à 6 et t désignant 0 ou 1. R'₆ représente un atome d'hydrogène, un radical alkyle en C₁-C₆ éventuellement substitué par un ou plusieurs groupements hydroxy.
      - Z₁ peut aussi représenter un radical divalent -S-, -SO-, -SO₂- lorsque R'₁ est un radical méthyle,
      - R'₁ et R'₂ représentent indépendamment :
         - un hydrogène
         - un radical alkyle en C₁-C₁₀, éventuellement substitué et éventuellement interrompu par un hétéroatome ou un groupement choisi parmi O, N, Si, S, SO, SO₂,
         - un halogène,
         - un radical SO₃H,
         - un cycle de 5 à 8 chaînons, substitué ou non, saturé, insaturé ou aromatique, renfermant éventuellement un ou plusieurs hétéroatomes ou groupements choisis parmi N, O, S, SO₂, -CO-, le cycle pouvant être cationique et/ou substitué par un radical cationique,
         - un groupement -N⁺R₁₇R₁₈R₁₉, R₁₇, R₁₈ et R₁₉ étant des alkyls linéaires ou ramifiés en C1-C5 éventuellement substituées par un ou plusieurs groupements hydroxy.
         lorsque Z₁ respectivement Z₂ représente une liaison covalente, alors R'₁ respectivement R'₂ peut aussi représenter un radical :
         - alkylcarbonyle en C₁-C₆,éventuellement substitué
            - -O-CO-R, -CO-O-R, NR-CO-R' ou -CO-NRR' dans lesquels R et R' représentent indépendamment un atome d'hydrogène ou un radical alkyle en C₁-C₆ éventuellement substitué,
      - R'₃, R'₄ et R'₅, identiques ou différents, représentent
         - un atome d'hydrogène,
         - un radical hydroxyle,
         - un radical alcoxy en C₁-C₆,
         - un radical alkylthio en C₁-C₆,
         - un radical amino,
         - un radical monoalkylamino,
         - un radical dialkylamino en C₁-C₆ dans lequel les radicaux alkyles peuvent former avec l'atome d'azote auquel ils sont rattachés un hétérocycle de 5 à 8 chaînons, saturé, insaturé, aromatique ou non, pouvant renfermer un ou plusieurs hétéroatomes ou groupements choisis parmi N, O, S, SO₂, CO, l'hétérocycle pouvant être cationique, et/ou substitué par un radical cationique,
         - un radical alkylcarbonyle en C₁-C₆, éventuellement substitué,
         - un radical -O-CO-R, -CO-O-R, NR-CO-R' ou -CO-NRR' avec R et R' tels que définis précédemment,
         - un halogène,
         - un radical -NHSO₃H,
         - un radical alkyle en C₁-C₄ éventuellement substitué,
         - un cycle carboné saturé, insaturé ou aromatique, éventuellement substitué.
         - R'₃, R'₄ et R'₅, peuvent former deux à deux un cycle partiellement saturé ou non,
      - X représente un ion ou groupe d'ions permettant d'assurer l'électronégativité du dérivé de formule (II),
   avec la condition qu'au moins un des groupements R'₁ et R'₂ représente un radical cationique,
   le procédé de coloration étant mis en oeuvre en l'absence de tout agent oxydant chimique.

Par oxydant chimique, on entend au sens de la présente invention tout composé chimique oxydant, liquide ou solide à la température de 25 °C et à la pression atmosphérique (10⁵ Pa), ajouté dans la composition tinctoriale. Ceci exclue l'oxygène de l'air.

Le procédé de la présente invention permet en particulier d'obtenir une coloration de fibres kératiniques qui convient pour une utilisation en coloration d'oxydation et permet d'obtenir une coloration aux nuances variées, puissantes, esthétiques, peu sélectives et résistant bien aux diverses agressions que peuvent subir les cheveux tels que les shampooings, la lumière, la sueur et les déformations permanentes. Le procédé de l'invention permet en particulier d'obtenir une coloration puissante et variée en l'absence de tout agent oxydant chimique ce qui permet d'obtenir des colorations résistantes sans dégradation des fibres kératiniques.

En effet, de façon surprenante, les pyazolopyridines utiles dans la présente invention permettent d'obtenir sans peroxyde d'hydrogène et uniquement en présence de l'oxygène de l'air des colorations très intenses des cheveux.

Il est à noter que dans ce qui va suivre, et à moins d'une autre indication, les bornes d'un domaine de valeurs sont comprises dans ce domaine.

Dans les composés de formule (I) et (II) ci-dessus, et sauf autre indication l'expression alkyle utilisée pour les radicaux alkyle ainsi que pour les groupements comportant une partie alkyle, signifie une chaîne carbonée, linéaire ou ramifiée, comportant de 1 à 4 atomes de carbone, substituée ou non substituée par un ou plusieurs hétérocycles, ou par un ou plusieurs groupements phényle ou par un ou plusieurs groupes choisis parmi les atomes d'halogène tel le chlore, le brome, l'iode et le fluor ; les radicaux hydroxyle, alcoxyle, amino, carbonyle, carboxamido, sulfonyle, -CO₂H, -SO₃H, -PO₃H₂, -PO₄H₂, -NHSO₃H, sulfonamide, monoalkyl(C₁-C₄)amino, trialkyl(C₁-C₄)ammonium, ou bien encore par un radical dialkyl(C₁-C₄)amino dans lequel les deux groupements alkyle peuvent former, conjointement avec l'atome d'azote dudit groupement dialkyl(C₁-C₄)amino auquel ils sont liés, un cycle pouvant être interrompu par un ou plusieurs atomes d'azote, d'oxygène ou de soufre.

De même, selon l'invention, l'expression alcoxy utilisée pour les radicaux alcoxy ainsi que pour les groupements comportant une partie alcoxy, signifie une chaîne O-carbonée, linéaire ou ramifiée, comportant de 1 à 4 de carbone, substituée ou non substituée par un ou plusieurs groupes choisis parmi les hétérocycles ; les atomes d'halogène tel le chlore, le brome, l'iode et le fluor ; les radicaux hydroxyle, amino, carbonyle, carboxamido, sulfonyle, -CO₂H, -SO₃H, -PO₃H₂, -PO₄H₂, -NHSO₃H, sulfonamide, monoalkyl(C₁-C₄)amino, trialkyl(C₁-C₄)ammonium, ou bien encore par un radical dialkyl(C₁-C₄)amino dans lequel les deux groupements alkyle peuvent former, conjointement avec l'atome d'azote dudit groupement dialkyl(C₁-C₄)amino auquel ils sont liés, un cycle pouvant être interrompu par un ou plusieurs atomes d'azote, d'oxygène ou de soufre.

Selon l'invention, on entend par hétérocycle, un cycle aromatique ou non contenant 5, 6, 7 ou 8 sommets, et de 1 à 3 hétéroatomes choisis parmi les atomes d'azote, de soufre et d'oxygène. Ces hétérocycles peuvent être condensés sur d'autres hétérocycles ou sur un groupement phényle. Ils peuvent être substitués par un atome d'halogène ; un radical (C₁-C₄)alkyle ; un radical (C₁-C₄)alkoxy ; un radical hydroxyle ; un radical amino ; un radical (C₁-C₄)alkylamino ; di(C₁-C₄) alkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre. Ces hétérocycles peuvent, en outre, être quaternisés par un radical (C₁-C₄)alkyle.

Parmi ces hétérocycles éventuellement condensés, on peut notamment citer à titre d'exemple les cycles : thiadiazole, triazole, isoxazole, oxazole, azaphosphole, thiazole, isothiazole, imidazole, pyrazole, triazine, thiazine, pyrazine, pyridazine, pyrimidine, pyridine, diazépine, oxazépine, benzotriazole, benzoxazole, benzimidazole, benzothiazole, morpholine, pipéridine, pipérazine, azétidine, pyrrolidine, aziridine, 3-(2-hydroxyéthyl)benzothiazol-3-ium, et 1-(2-hydroxyéthyl)-pyridinium.

Selon l'invention, on entend par phényle, un radical phényle non substitué ou substitué par un ou plusieurs radicaux cyano, carbonyle, carboxamido, sulfonyle, - CO₂H, -SO₃H, -PO₃H₂, -PO₄H₂, hydroxyle, amino, monoalkyl(C₁-C₄)amino, ou dialkyl(C₁-C₄)amino dans lequel les deux groupements alkyle peuvent former, conjointement avec l'atome d'azote dudit groupement dialkyl(C₁-C₄)amino auquel ils sont liés, un cycle pouvant être interrompu par un ou plusieurs atomes d'azote, d'oxygène ou de soufre.

Parmi les groupements 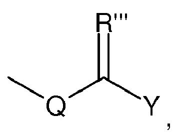 on peut notamment citer les groupement acétamide, diméthylurée, O-méthylcarbamate, méthylcarbonate, N-diméthylcarbamate et les esters.

Parmi les composés de formule (I) ci-dessus, on préfère les 3-amino pyrazolo-[1,5-a]-pyridines répondant à la sous-formule suivante, et leurs sels d'addition avec un acide ou avec une base : 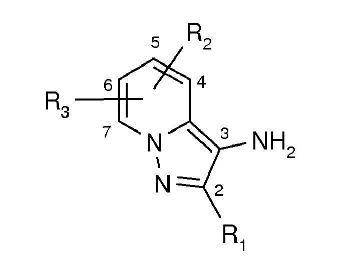 dans laquelle :
R₁, R₂, R₃, identiques ou différents, représentent un atome d'hydrogène ou d'halogène ; un radical hydroxyle ; un radical (C₁-C₄)alkyle ; un radical (C₁-C₄)alkylthio ; un radical (C₁-C₄)alkoxy ; un radical -NHSO₃H ; un radical amino ; un radical (C₁-C₄)alkylamino ; un radical di(C₁-C₄)alkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre ; un hétérocycle tel que défini précédemment ; un radical sulfonamide, un radical carbonyle, un radical alcoxy(C₁-C₄)carbonyle, un radical carboxamido, ou un groupement de formule : 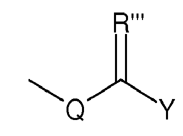 dans lequel R"' représente un atome d'oxygène ou d'azote, Q représente un atome d'oxygène, un groupement NH ou NH(C₁-C₄)alkyle, et Y représente un radical hydroxyle, amino, alkyle en C₁-C₄, (C₁-C₄)alkoxy, (C₁-C₄)alkylamino, ou di(C₁-C₄)alkylamino.

Parmi les 3-amino pyrazolo-[1,5-a]-pyridines de formule (I), utilisables à titre de base d'oxydation dans les compositions tinctoriales conformes à l'invention, on peut notamment citer :
- la pyrazolo[1,5-a]pyridin-3-ylamine ;
- la 2-acétylamino pyrazolo-[1,5-a] pyridin-3-ylamine ;
- la 2-morpholin-4-yl-pyrazolo[1 ,5-a]pyridin-3-ylamine ;
- l'acide 3-amino-pyrazolo[1 ,5-a]pyridin-2-carboxylique ;
- la 2-méthoxy-pyrazolo[1,5-a]pyridine-3-ylamino ;
- le (3-amino-pyrazolo[1 ,5-a]pyridine-7-yl)-méthanol ;
- le 2-(3-amino-pyrazolo[1 ,5-a]pyridine-5-yl)-éthanol ;
- le 2-(3-amino-pyrazolo[1,5-a]pyridine-7-yl)-éthanol ;
- le (3-amino-pyrazolo[1 ,5-a]pyridine-2-yl)-méthanol ;
- la 3,6-diamino-pyrazolo[1,5-a]pyridine ;
- la 3,4-diamino-pyrazolo[1,5-a]pyridine ;
- la pyrazolo[1,5-a]pyridine-3,7-diamine ;
- la 7-morpholin-4-yl-pyrazolo[1 ,5-a]pyridin-3-ylamine ;
- la pyrazolo[1,5-a]pyridine-3,5-diamine ;
- la 5-morpholin-4-yl-pyrazolo[1 ,5-a]pyridin-3-ylamine ;
- le 2-[(3-amino-pyrazolo[1 ,5-a]pyridin-5-yl)-(2-hydroxyéthyl)-amino]-éthanol ;
- le 2-[(3-amino-pyrazolo[1,5-a]pyridin-7-yl)-(2-hydroxyéthyl)-amino]-éthanol ;
- la 3-amino-pyrazolo[1,5-a]pyridine-5-ol ;
- la 3-amino-pyrazolo[1,5-a]pyridine-4-ol ;
- la 3-amino-pyrazolo[1 ,5-a]pyridine-6-ol ;
- la 3-amino-pyrazolo[1,5-a]pyridine-7-ol ;
- la 2-methoxy-6,7-dimethylpyrazolo[1,5-a]pyridin-3-amine ;
- la 2-[(3-aminopyrazolo[1,5-a]pyridin-2-yl)oxy]éthanol ;
- la 4-ethyl-2-methoxy-7-methylpyrazolo[1,5-a]pyridin-3-amine hydrochloride ;
- la 1-(3-aminopyrazolo[1,5-a]pyridin-2-yl)pyrrolidin-3-ol ;
- la 2,2'-[(3-aminopyrazolo[1 ,5-a]pyridin-2-yl)imino]diethanol ;
- la 2-[(3-aminopyrazolo[1,5-a]pyridin-2-yl)amino]ethanol ;
- la N2-(2-pyridin-3-ylethyl)pyrazolo[1 ,5-a]pyridine-2,3-diamine ;
et leurs d'addition avec un acide ou avec une base.

Pour leur grande majorité, les 3-amino pyrazolo-[1,5-a]-pyridines de formule (I) sont des composés connus dans le domaine pharmaceutique, et sont décrites notamment dans le brevet US 5,457,200. Ces composés peuvent être préparés selon des méthodes de synthèse bien connues dans la littérature et telles que décrites par exemple dans le brevet US 5,457,200.

Par cycle ou hétérocycle cationique, on entend un cycle contenant un ou plusieurs groupements ammoniums quaternaires.

A titre d'exemple de radicaux du type -N⁺R₁₇R₁₈R₁₉, on peut citer les radicaux triméthylammonium, triéthylammonium, diméthyl-éthyl ammonium, diéthylméthylammonium, diisopropylméthylammonium, diéthyl-propyl ammonium, betahydroxyéthyl diéthyl ammonium, di (betahydroxyethyl)methylammonium, tri (betahydroxyethyl) ammonium.

A titre d'exemple d'hétérocycle cationique, on peut citer les hétérocycles imidazoliums, pyridiniums, pipéraziniums, pyrrolidiniums, morpholiniums, pyrimidiniums, thiazoliums, benzimidazoliums, benzothiazoliums, oxazoliums, benzotriazoliums, pyrazoliums, triazoliums, benzoxazoliums.

A titre d'exemple d'hétérocycle cationique, on peut citer les imidazoliums, les pyridiniums, les pipéraziniums, les pyrrolidiniums, les morpholiniums , les pyrimidiniums, les thiazoliums, les benzimidazoliums, les benzothiazoliums, les oxazoliums, les benzotriazoliums, les pyrazoliums, les triazoliums, les benzoxazoliums.

Les composés de formule (II) peuvent être éventuellement salifiés par des acides minéraux forts tels que par exemple HCI, HBr, HI, H₂SO₄, H₃PO₄, ou des acides organiques tels que, par exemple, l'acide acétique, lactique, tartrique, citrique ou succinique, benzènesulfonique, para-toluènesulfonique, formique, méthanesulfonique.

S'ils possèdent des groupements anioniques tels que les groupements -CO₂H, -SO₃H, -PO₃H₂, -PO₄H₂, les composés de formules (I) peuvent être salifiés par des hydroxydes de métaux alcalins ou alcalinoterreux tels que la soude ou la potasse, par l'ammoniaque, par les amines organiques.

Ils peuvent aussi être sous forme de solvates par exemple un hydrate ou un solvate d'alcool linéaire ou ramifié tel que l'éthanol ou l'isopropanol.

Dans le cadre de l'invention, on entend par dérivé de formule (II) toutes formes mésomères ou isomères.

A titre d'exemples de dérivés de formule (II), on peut citer les composés suivants dans lesquels X⁻ est tel que défini précédemment :

| |
|---|
| 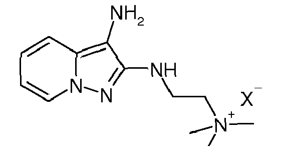 |
| Sel de [2-(3-Amino-pyrazolo[1,5-a]pyridin-2-ylamino)-ethyl]-trimethyl-ammonium |
| 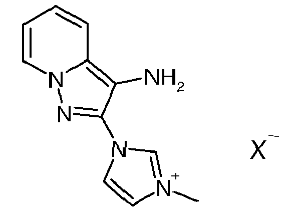 |
| Sel de 3-(3-Amino-pyrazolo[1,5-a]pyridin-2-yl)-1-methyl-3H-imidazol-1-ium |
| 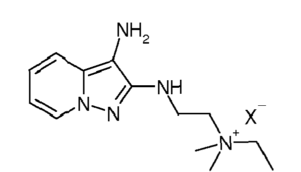 |
| Sel de [2-(3-Amino-pyrazolo[1,5-a]pyridin-2-ylamino)-ethyl]-ethyl-dimethyl-ammonium |
| 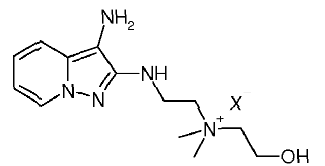 |
| Sel de [2-(3-Amino-pyrazolo[1,5-a]pyridin-2-ylamino)-ethyl]-(2-hydroxy-ethyl)-dimethyl-ammonium |
| 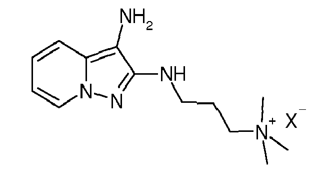 |
| Sel de [3-(3-Amino-pyrazolo[1,5-a]pyridin-2-ylamino)-propyl]-trimethyl-ammonium |
| 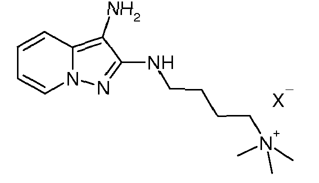 |
| Sel de [4-(3-Amino-pyrazolo[1 ,5-a]pyridin-2-ylamino)-butyl]-trimethyl-ammonium |
| 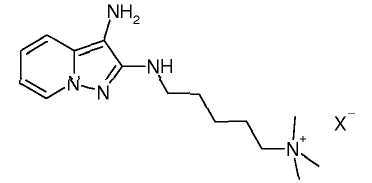 |
| Sel de [5-(3-Amino-pyrazolo[1,5-a]pyridin-2-ylamino)-pentyl]-trimethyl-ammonium |
| 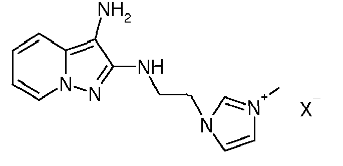 |
| Sel de 3-[2-(3-Amino-pyrazolo[1,5-a]pyridin-2-ylamino)-ethyl]-1-methyl-3H-imidazol-1-ium |
| 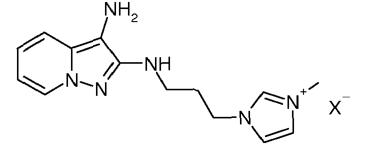 |
| Sel de 3-[3-(3-Amino-pyrazolo[1,5-a]pyridin-2-ylamino)-propyl]-1-methyl-3H-imidazol-1-ium |
| 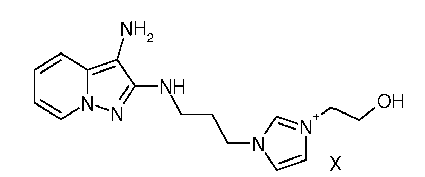 |
| Sel de 3-[3-(3-Amino-pyrazolo[1,5-a]pyridin-2-ylamino)-propyl]-1-(2-hydroxy-ethyl)-3H-imidazol-1-ium |
| 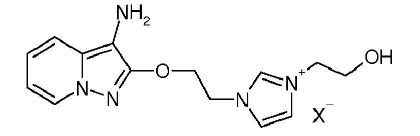 |
| Sel de 3-[2-(3-Amino-pyrazolo[1,5-a]pyridin-2-yloxy)-ethyl]-1-(2-hydroxy-ethyl)-3H-imidazol-1-ium |
| 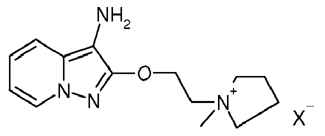 |
| Sel de 1-{2-[(3-aminopyrazolo[1,5-a]pyridin-2-yl)oxy]ethyl}-1-methylpyrrolidinium |
| 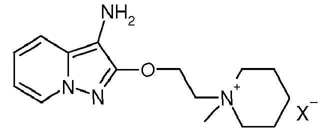 |
| Sel de 1-{2-[(3-aminopyrazolo[1,5-a]pyridin-2-yl)oxy]ethyl}-1-methylpiperidinium |
| 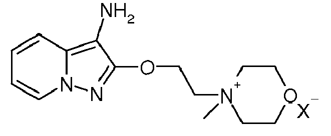 |
| Sel de 4-{2-[(3-aminopyrazolo[1,5-a]pyridin-2-yl)oxy]ethyl}-4-methylmorpholin-4-ium |
| 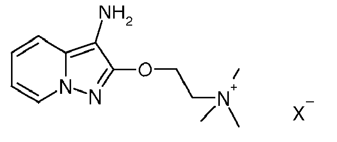 |
| Sel de {2-[(3-aminopyrazolo[1,5-a]pyridin-2-yl)oxy]ethyl}-trimethyl-ammonium |
| 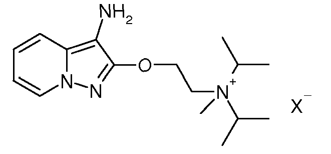 |
| Sel de {2-[(3-aminopyrazolo[1,5-a]pyridin-2-yl)oxy]ethyl}-diisopropyl-methyl-ammonium |
| 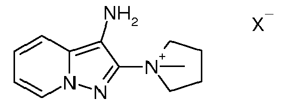 |
| Sel de 1-(3-aminopyrazolo[1,5-a]pyridin-2-yl)-1-methylpyrrolidinium |
| 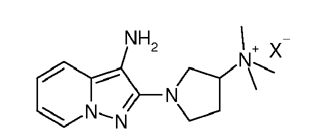 |
| Sel de [1-(3-Amino-pyrazolo[1,5-a]pyridin-2-yl)-pyrrolidin-3-yl]-trimethyl-ammonium |
| 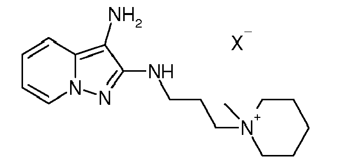 |
| Sel de 1-[3-(3-Amino-pyrazolo[1,5-a]pyridin-2-ylamino)-propyl]-1-methylpiperidinium |
| 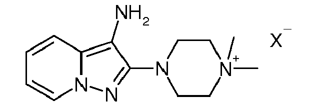 |
| Sel de 4-(3-Amino-pyrazolo[1,5-a]pyridin-2-yl)-1,1-dimethyl-piperazin-1-ium |
| 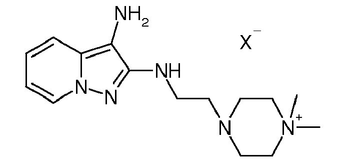 |
| Sel de 4-[2-(3-Amino-pyrazolo[1,5-a]pyridin-2-ylamino)-ethyl]-1,1-dimethyl-piperazin-1-ium |
| 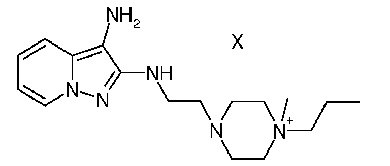 |
| Sel de 4-[2-(3-Amino-pyrazolo[1,5-a]pyridin-2-ylamino)-ethyl]-1-methyl-1-propyl-piperazin-1-ium |
| 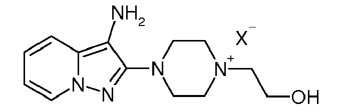 |
| Sel de 4-(3-Amino-pyrazolo[1,5-a]pyridin-2-yl)-1-(2-hydroxy-ethyl)-piperazin-1-ium |
| 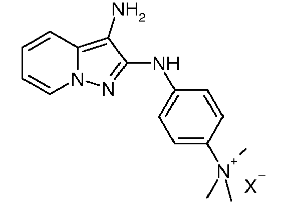 |
| Sel de [4-(3-Amino-pyrazolo[1,5-a]pyridin-2-ylamino)-phenyl]-trimethyl-ammonium |
| 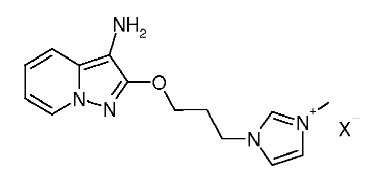 |
| Sel de 3-[3-(3-Amino-pyrazolo[1,5-a]pyridin-2-yloxy)-propyl]-1-methyl-3H-imidazol-1-ium |
| 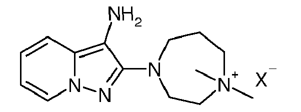 |
| Sel de 4-(3-Amino-pyrazolo[1,5-a]pyridin-2-yl)-1,1-dimethyl-[1,4]diazepan-1-ium |
| 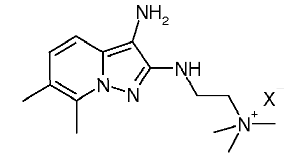 |
| Sel de [2-(3-Amino-6,7-dimethyl-pyrazolo[1,5-a]pyridin-2-ylamino)-ethyl]-trimethyl-ammonium |
| 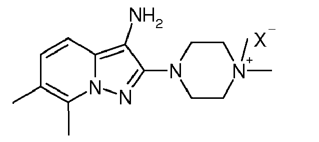 |
| Sel de 4-(3-Amino-6,7-dimethyl-pyrazolo[1,5-a]pyridin-2-yl)-1,1-dimethyl-piperazin-1-ium |
| 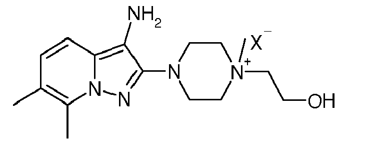 |
| Sel de 4-(3-Amino-6,7-dimethyl-pyrazolo[1,5-a]pyridin-2-yl)-1-(2-hydroxy-ethyl)-1-methyl-piperazin-1-ium |
| 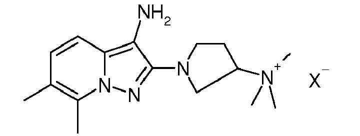 |
| Sel de [1-(3-Amino-6,7-dimethyl-pyrazolo[1,5-a]pyridin-2-yl)-pyrrolidin-3-yl]-trimethyl-ammonium |
| 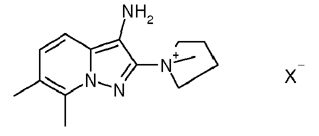 |
| Sel de 1-(3-amino-6,7-dimethylpyrazolo[1,5-a]pyridin-2-yl)-1-methylpyrrolidinium |
| 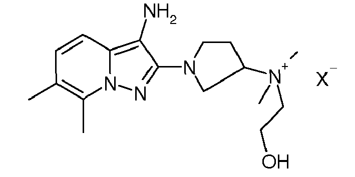 |
| Sel de [1-(3-Amino-6,7-dimethyl-pyrazolo[1,5-a]pyridin-2-yl)-pyrrolidin-3-yl]-(2-hydroxy-ethyl)-dimethyl-ammonium |
| 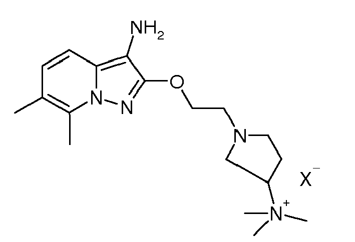 |
| Sel de {1-[2-(3-Amino-6,7-dimethyl-pyrazolo[1,5-a]pyridin-2-yloxy)-ethyl]-pyrrolidin-3-yl}-trimethyl-ammonium |
| 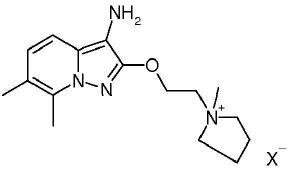 |
| Sel de 1-{2-[(3-amino-6,7-dimethylpyrazolo[1,5-a]pyridin-2-yl)oxy]ethyl}-1-methyl pyrrolidinium |
| 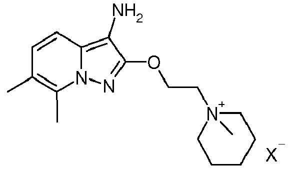 |
| Sel de 1-{2-[(3-amino-6,7-dimethylpyrazolo[1,5-a]pyridin-2-yl)oxy]ethyl}-1-methylpiperidinium |
| 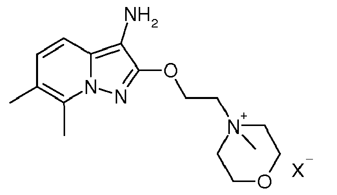 |
| Sel de 4-{2-[(3-amino-6,7-dimethylpyrazolo[1,5-a]pyridin-2-yl)oxy]ethyl}-4-methylmoraholin-4-ium |
| 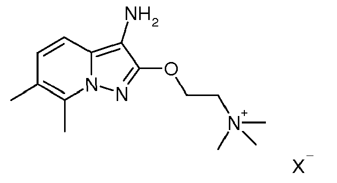 |
| Sel de {2-[(3-amino-6,7-dimethylpyrazolo[1,5-a]pyridin-2-yl)oxy]ethyl}-trimethyl-ammonium |
| 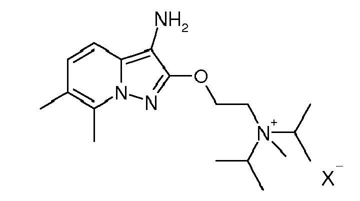 |
| Sel de {2-[(3-amino-6,7-dimethylpyrazolo[1,5-a]pyridin-2-yl)oxy]ethyl}-diisopropyl-methyl-ammonium |
| 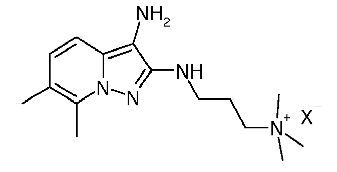 |
| Sel de [3-(3-Amino-6,7-dimethyl-pyrazolo[1,5-a]pyridin-2-ylamino)-propyl]-trimethyl-ammonium |
| 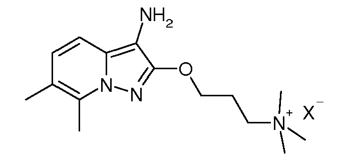 |
| Sel de [3-(3-Amino-6,7-dimethyl-pyrazolo[1,5-a]pyridin-2-yloxy)-propyl]-trimethyl-ammonium |
| 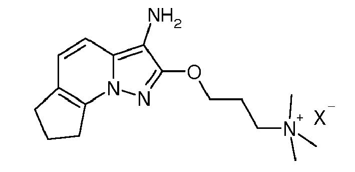 |
| Sel de [3-(3-Amino-7,8-dihydro-6H-cyclopenta[e]pyrazolo[1,5-a]pyridin-2-yloxy)-propyl]-trimethyl-ammonium |
| 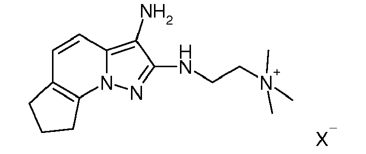 |
| Sel de {2-[(3-amino-7,8-dihydro-6H-cyclopenta[e]pyrazolo[1,5-a]pyridin-2-yl)amino]-ethyl}-trimethyl-ammonium |
| 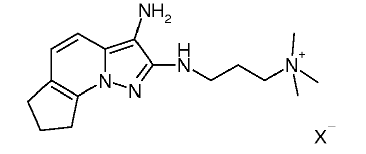 |
| Sel de {3-[(3-amino-7,8-dihydro-6H-cyclopenta[e]pyrazolo[1,5-a]pyridin-2-yl)amino]-propyl}-trimethyl-ammonium |
| 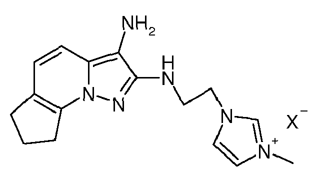 |
| Sel de 1-{2-[(3-amino-7,8-dihydro-6H-cyclopenta[e]pyrazolo[1,5-a]pyridin-2-yl)amino]ethyl}-3-methyl-1H-imidazol-3-ium |
| 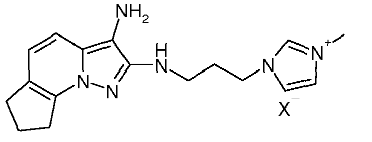 |
| Sel de 1-{3-[(3-amino-7,8-dihydro-6H-cyclopenta[e]pyrazolo[1,5-a]pyridin-2-yl)aminolpropyl}-3-methyl-1 H-imidazol-3-ium |
| 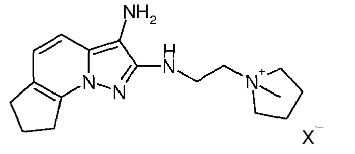 |
| Sel de 1-{2-[(3-amino-7,8-dihydro-6H-cyclopenta[e]pyrazolo[1,5-a]pyridin-2-yl)amino]ethyl}-1-methylpyrrolidinium |
| 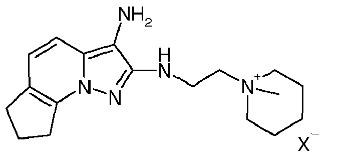 |
| Sel de 1-{2-[(3-amino-7,8-dihydro-6H-cyclopenta[e]pyrazolo[1,5-a]pyridin-2-yl)amino]ethyl}-1-methylperidinium |
| 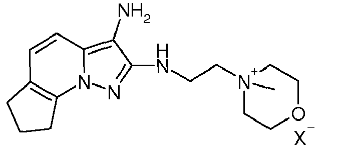 |
| Sel de 4-{2-[(3-amino-7,8-dihydro-6H-cyclopenta[e]pyrazolo[1,5-a]pyridin-2-yl)amino]ethyl}-4-methylmorpholin-4-ium |
| 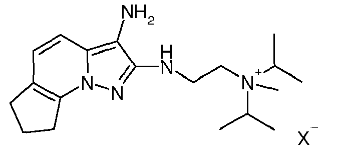 |
| Sel de {2-[(3-amino-7,8-dihydro-6H-cyclopenta[e]pyrazolo[1,5-a]pyridin-2-yl)amino]ethyl}-diisopropyl-methyl-ammonium |
| 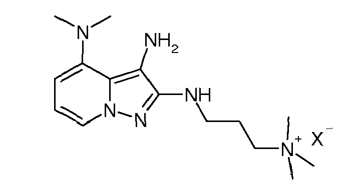 |
| Sel de [3-(3-Amino-4-dimethylamino-pyrazolo[1,5-a]pyridin-2-ylamino)-propyl]-trimethyl-ammonium |
| 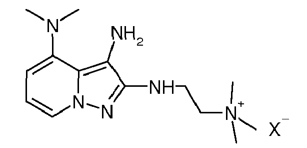 |
| Sel de [2-(3-Amino-4-dimethylamino-pyrazolo[1,5-a]pyridin-2-ylamino)-ethyl]-trimethyl-ammonium |
| 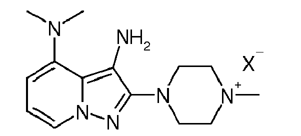 |
| Sel de 4-(3-Amino-4-dimethylamino-pyrazolo[1,5-a]pyridin-2-yl)-1-methyl-piperazin-1-ium |
| 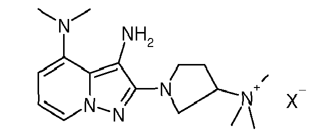 |
| Sel de [1-(3-Amino-4-dimethylamino-pyrazolo[1,5-a]pyridin-2-yl)-pyrrolidin-3-yl]-trimethyl-ammonium |
| 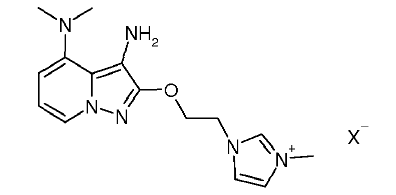 |
| Sel de 3-[2-(3-Amino-4-dimethylamino-pyrazolo[1,5-a]pyridin-2-yloxy)-ethyl]-1-methyl-3H-imidazol-1-ium |
| 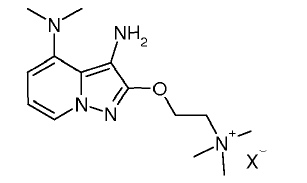 |
| Sel de [2-(3-Amino-4-dimethylamino-pyrazolo[1,5-a]pyridin-2-yloxy)-ethyl]-trimethyl-ammonium |
| 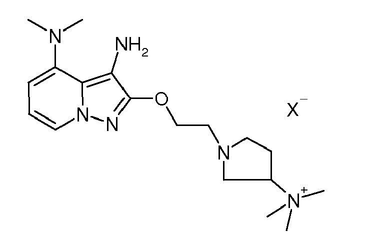 |
| Sel de {1-[2-(3-Amino-4-dimethylamino-pyrazolo[1,5-a]pyridin-2-yloxy)-ethyl]-pyrrolidin-3-yl}-trimethyl-ammonium |
| 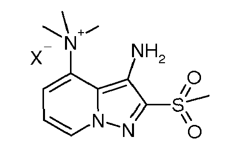 |
| Sel de (3-Amino-2-methanesulfonyl-pyrazolo[1,5-a]pyridin-4-yl)-trimethyl-ammonium |
| 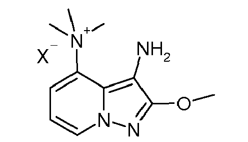 |
| Sel de (3-Amino-2-methoxy-pyrazolo[1,5-a]pyridin-4-yl)-trimethyl-ammonium |

La nature du contre-ion n'est pas déterminante sur le pouvoir tinctorial des composés de formule (II).

Lorsque R'₁ ou R'₂ désignent un hétérocycle, cet hétérocycle est de préférence un hétérocycle cationique ou un hétérocycle substitué par un radical cationique. A titre d'exemple, on peut citer les imidazoles substitués par un radical ammonium quaternaire ou les imidazoliums, les pipérazines substitués par un radical ammonium quaternaire ou les pipéraziniums, les pyrrolidines substitués par un radical ammonium quaternaire ou les pyrrolidiniums, les diazépanes substitués par un radical ammonium quaternaire ou les diazépaniums.

Selon un mode de réalisation différent, R'₁ ou R'₂ représentent un groupement - N⁺R₁₇R₁₈R₁₉, R₁₇, R₁₈ et R₁₉ étant des alkyls linéaires ou ramifiés en C1-C5 éventuellement substituées par un ou plusieurs groupements hydroxy, tel que trialkylammonium, tri(hydroxyalkyl)ammonium, hydroxyalkyl-dialkyl-ammonium ou di(hydroxyalkyl)alkylammonium.

Les radicaux R'₃, R'₄ et R'₅ indépendamment peuvent être un atome d'hydrogène, un radical alkyle en C₁-C₄ pouvant être substitué. A titre d'exemple, on peut citer les radicaux méthyle, éthyle, hydroxy éthyle, amino éthyle, propyle, butyle. Selon un mode de réalisation particulier, R'₃, R'₄ et R'₅ représentent indépendamment un atome d'hydrogène, un radical alkyle en C₁-C₄.

Selon un mode de réalisation particulier, R'₄ et R'₅ forment ensemble un cycle partiellement saturé ou insaturé à 5 ou 8 chaînons, notamment un cyclopentène ou cyclohexène, éventuellement substitué.

Selon un mode de réalisation particulier, le composé de formule (II) correspond à la formule (Il') suivante : 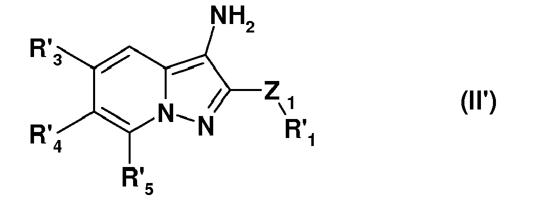 dans laquelle Z₁, R'₁, R'₃, R'₄ et R'₅ sont tels que définis précédemment.

Selon un mode de réalisation particulier de cette formule, Z₁ représente une liaison covalente, un radical -NR'₆(CH₂)_{q}- ou un radical -O(CH₂)ₚ- et R'₁ est un radical cationique

La ou les bases d'oxydation de l'invention sont en général présentes chacune en quantité comprise entre 0,001 à 10 % en poids environ du poids total de la composition tinctoriale, de préférence entre 0,005 et 6 %.

La composition tinctoriale de l'invention peut contenir un ou plusieurs coupleurs conventionnellement utilisés pour la teinture de fibres kératiniques. Parmi ces coupleurs, on peut notamment citer les méta-phénylènediamines, les métaaminophénols, les méta-diphénols, les coupleurs naphtaléniques, les coupleurs hétérocycliques et leur sels d'addition.

A titre d'exemple de coupleur, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 2,4-dichloro-3-aminophenol, le 5-amino-4-chloro-o-cresol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 1,5-dihydroxy naphtalene, le 2,7-naphthalenediol, le 1-acetoxy-2-methylnaphthalene, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, les 2,6-dihydroxy-3-4-dimethyl pyridine, le 3-amino-2-methylamino-6-methoxypyridine, le 1-N-(β-hydroxyéthyl)amino-3,4-méthylène dioxybenzène, le 2,6-bis-(β-hydroxyéthylamino)toluène, le 3-methyl-1-phenyl-5-pyrazolone phenyl methyl pyrazolone et leurs sels d'addition avec un acide.

La composition de la présente invention peut en outre comprendre une ou plusieurs bases d'oxydation additionnelles classiquement utilisées en teinture d'oxydation différentes des bases d'oxydation de formule (I) et (II). A titre d'exemple, ces bases d'oxydation additionnelles sont choisies parmi les para-phénylènediamines autres que celles décrites précédemment, les bis-phénylalkylènediamines, les para-aminophénols, les bis-para-aminophénols, les ortho-aminophénols, les bases hétérocycliques différentes des bases d'oxydation de formules (I) et (II) et leurs sels d'addition.

Parmi les para-phénylènediamines, on peut citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylène-diamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

On peut aussi citer les diaminopyrazolinones décrites dans la demande de brevet FR2886137 et en particulier la 2,3-diamino-6,7-dihydro-1H,5H-pyrazol-1-one et ses sels.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture comprend généralement de l'eau ou un mélange d'eau et au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques. De préférence, le milieux est alcalin (pH>7)

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 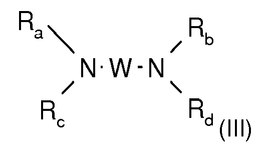 dans laquelle W est un reste propylène éventuellement substitué par un groupement hydroxyle ou un radical alkyle en C₁-C₄ Rₐ, R_{b}, P_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Dans le procédé de la présente invention, après un temps de pose de 1 à 60 minutes environ, de préférence 5 à 45 minutes environ, les fibres kératiniques sont généralement rincées, lavées au shampooing, rincées à nouveau puis séchées.

La composition prête à l'emploi qui est appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Dans le cadre de l'invention, le procédé de l'invention est mis en oeuvre en présence d'oxygène de l'air en l'absence d'autres agents oxydants chimiques, ce qui signifie que la couleur est obtenue en l'absence d'un agent oxydant chimique classiquement utilisé dans le domaine de la coloration par oxydation, c'est-à-dire en l'absence d'agents oxydants chimiques tels que par exemple le peroxyde d'hydrogène, le peroxyde d'urée, les bromates de métaux alcalins, les persels tels que les perborates et persulfates et les peracides.

Les exemples qui suivent servent à illustrer l'invention sans toutefois présenter un caractère limitatif.

### EXEMPLES

### EXEMPLE 1

Les compositions suivantes ont été préparées en introduisant dans le milieu décrit ci après les colorants suivants (quantité exprimée en mole) :

| En moles pour 100 g de composition | **B1** | **B2** | **C1** | **C2** | **C3** | **C4** |
|---|---|---|---|---|---|---|
| Composition A1 | 0.005 | | | | | |
| Composition A2 | | 0.005 | | | | |
| Composition A8 | 0.0025 | | 0.0025 | | | |
| Composition A9 | 0.0025 | | | 0.0025 | | |
| Composition A10 | 0.0025 | | | | 0.0025 | |
| Composition A11 | 0.0025 | | | | | 0.0025 |
| Composition A13 | | 0.0025 | 0.0025 | | | |
| Composition A14 | | 0.0025 | | 0.0025 | | |
| Composition A15 | | 0.0025 | | | 0.0025 | |
| Composition A16 | | 0.0025 | | | | 0.0025 |

| | | | | | | |
|---|---|---|---|---|---|---|
| B1= Chlorhydrate de chlorure de 2-[(3-aminopyrazolo[1,5-a]pyridin-2-yl)amino]-N,N,N-trimethylethanaminium B2 = Chlorhydrate de 2-[(3-aminopyrazolo[1,5-a]pyridin-2-yl)oxy]ethanol C1 = 1-METHYL-2-HYDROXY-4-AMINOBENZENE C2 = 2,4-DIAMINOPHENOXYETHANOL C3 = 2-AMINO-3-HYDROXYPYRIDINE C4 = 6-HYDROXYINDOLE | | | | | | |

| Milieu de coloration : | |
|---|---|
| Sulfite de sodium | 0.1 g |
| Lauryl ether sulfate de sodium oxyéthyléné (2.2 OE) à 30% en solution aqueuse | 1.0 g |
| Ethanol | 5.0 g |
| Ammoniaque à 20% de NH₃ | 4.0 g |
| Eau déminéralisée q.s.p | 100 g |

### Mode d'application

La formule est appliquée sur des cheveux gris naturels à 90% de cheveux blancs à raison de 30g pour 3 g de cheveux pendant 30mn sur plateau chauffant à 27 °C. Les cheveux sont ensuite rincés, lavés avec un shampooing standard et séchés

### Résultats

La coloration capillaire est évaluée de manière visuelle.

| **Compositions** | **Hauteur de ton** | **Reflet** |
|---|---|---|
| Composition A1 | Blond | Vert |
| Composition A2 | Blond très Clair | Doré |
| Composition A8 | Blond Foncé | Violine Chromatique |
| Composition A9 | Blond Clair | Naturel Irisé |
| Composition A10 | Blond | Bleu |
| Composition A11 | Blond Foncé | Naturel |
| Composition A13 | Blond Clair | Cuivré Irisé |
| Composition A14 | Blond Clair | Naturel Irisé |
| Composition A15 | Blond Clair | Naturel Cendré |
| Composition A16 | Blond Clair | Doré Cuivré |

Ces nuances sont tenaces et uniformes

### Exemple 2

On a préparé une composition comprenant le composé suivant en mélange avec C₂ en quantité molaire égale. On obtient ainsi une coloration bleu vert, tenace et uniforme. 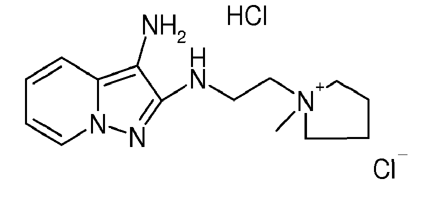 Chlorure de 1-{2-[(3-aminopyrazolo[1,5-a]pyridin-2-yl)amino]ethyl}-1-methylpyrrolidinium

### Exemple 3

On a préparé une composition comprenant le composé suivant en mélange avec C₂ en quantité molaire égale. On obtient ainsi une coloration bleu vert, tenace et uniforme. 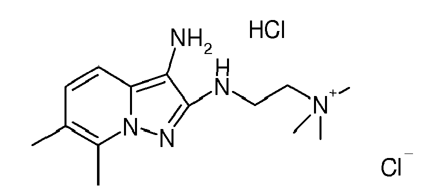 Clhorure de 2-[(3-amino-6,7-dimethylpyrazolo[1,5-a]pyridin-2-yl)amino]-N,N,N-trimethylethanaminium.

### Exemple 4

La composition I suivante a été préparée :

| | |
|---|---|
| Para-aminophenol (0.0025 mol) | 0.28 g |
| 1-Methyl-2-hydroxy-4-aminobenzène (0.0025 mol) | 0.31 g |
| Sulfite de sodium | 0.1 g |
| Lauryl ether sulfate de sodium oxyéthyléné (2.2 OE) à 30% en solution aqueuse | 1.0 g |
| Ethanol | 5.0 g |
| Ammoniaque à 20% de NH₃ | 4.0 g |
| Eau déminéralisée q.s.p | 100 g |

La composition II a été préparée :

| | |
|---|---|
| 2-[(3-Aminopyrazolo[1,5-a]pyridin-2-yl)oxy]ethanol, HCl (0.0025 mol) | 0.57 g |
| 1-Methyl-2-hydroxy-4-aminobenzène (0.0025 mol) | 0.31 g |
| Sulfite de sodium | 0.1 g |
| Lauryl ether sulfate de sodium oxyéthyléné (2.2 OE) à 30% en solution aqueuse | 1.0 g |
| Ethanol | 5.0 g |
| Ammoniaque à 20% de NH₃ | 4.0 g |
| Eau déminéralisée q.s.p | 100 g |

### Mode d'application

Les mélanges extemporanés ainsi ont été appliqués sur des cheveux gris à 90% blancs à raison de 30g pour 3 g de cheveux pendant 30mn sur plateau chauffant à 27°C.
Les cheveux ont ensuite été rincés, lavés avec un shampooing standard et séchés

### Résultats

La coloration capillaire a été évaluée de manière visuelle.

| **Compositions** | **Hauteur de ton 90% BN** | **Reflet 90% BN** |
|---|---|---|
| Composition I | Blond très Clair | Doré cuivré |
| Composition II | Blond Clair | Cuivré Irisé |

La composition II selon l'invention donne une coloration plus intense et plus chromatique que I.

## Revendications

1. Procédé de coloration des cheveux comprenant l'application sur les cheveux d'une composition comprenant, dans un milieu de teinture approprié, au moins une base d'oxydation aminopyrazolopyridine choisie parmi les bases de formule (I) ou (II), 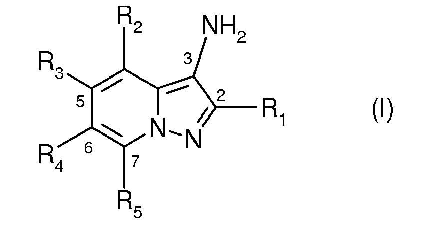 dans laquelle :
- R₁, R₂, R₃, R₄ et R₅, identiques ou différents, représentent un atome d'hydrogène ou d'halogène ; un radical -NHSO₃H ; un radical hydroxyle ; un radical (C₁-C₄)alkyle ; un radical (C₁-C₄)alkoxy ; un radical (C₁-C₄)alkylthio ; mono(C₁-C₄)alkylamino ; un radical di(C₁-C₄)alkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre ; un hétérocycle ; un radical nitro ; un radical phényle ; un radical carbonyle ; un radical alkoxy(C₁-C₄)carbonyle ; un radical carboxamido ; un radical cyano ; un radical amino ; un radical sulfonyle; un radical -CO₂H, un radical -SO₃H ; un radical -PO₃H₂ ; un radical -PO₄H₂; ou un groupement : 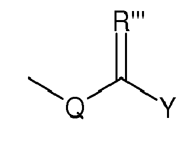 dans lequel R"' représente un atome d'oxygène ou d'azote, Q représente un atome d'oxygène, un groupement NH ou NH(C₁-C₄)alkyle, et Y représente un radical hydroxyle, amino, alkyle en C₁-C₄, (C₁-C₄)alkoxy, (C₁-C₄)alkylamino, ou di(C₁-C₄)alkylamino. 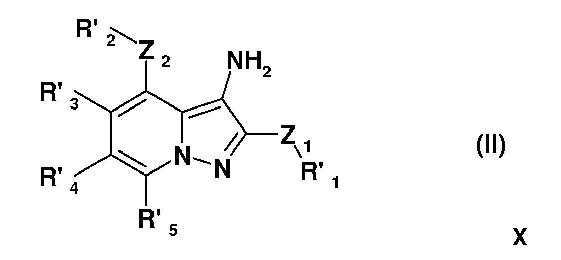 dans laquelle
• Z₁ et Z₂ représentent indépendamment
- une liaison covalente simple,
- un radical divalent choisi parmi
- un radical -O(CH₂)ₚ-, p désignant un nombre entier allant de 0 à 6
- un radical -NR'₆(CH₂)_{q}(C₆H₄)ₜ-, q désignant un nombre entier allant de 0 à 6 et t désignant 0 ou 1. R'₆ représente un atome d'hydrogène, un radical alkyle en C₁-C₆ éventuellement substitué par un ou plusieurs groupements hydroxy.
• Z₁ peut aussi représenter un radical divalent -S-, -SO-, -SO₂- lorsque R'₁ est un radical méthyle,
• R'₁ et R'₂ représentent indépendamment :
- un hydrogène
- un radical alkyle en C₁-C₁₀, éventuellement substitué et éventuellement interrompu par un hétéroatome ou un groupement choisi parmi O, N, Si, S, SO, SO₂,
- un halogène,
- un radical SO₃H,
- un cycle de 5 à 8 chaînons, substitué ou non, saturé, insaturé ou aromatique, renfermant éventuellement un ou plusieurs hétéroatomes ou groupements choisis parmi N, O, S, SO₂, -CO-, le cycle pouvant être cationique et/ou substitué par un radical cationique,
- un groupement -N⁺R₁₇R₁₈R₁₉, R₁₇, R₁₈ et R₁₉ étant des alkyls linéaires ou ramifiés en C₁-C₅ éventuellement substituées par un ou plusieurs groupements hydroxy.
lorsque Z₁ respectivement Z₂ représente une liaison covalente, alors R'₁ respectivement R'₂ peut aussi représenter un radical :
- alkylcarbonyle en C₁-C₆, éventuellement substitué
- -O-CO-R, -CO-O-R, NR-CO-R' ou -CO-NRR' dans lesquels R et R' représentent indépendamment un atome d'hydrogène ou un radical alkyle en C₁-C₆ éventuellement substitué,
• R'₃, R'₄ et R'₅, identiques ou différents, représentent
- un atome d'hydrogène,
- un radical hydroxyle,
- un radical alcoxy en C₁-C₆,
- un radical alkylthio en C₁-C₆,
- un radical amino,
- un radical monoalkylamino,
- un radical dialkylamino en C₁-C₆ dans lequel les radicaux alkyles peuvent former avec l'atome d'azote auquel ils sont rattachés un hétérocycle de 5 à 8 chaînons, saturé, insaturé, aromatique ou non, pouvant renfermer un ou plusieurs hétéroatomes ou groupements choisis parmi N, O, S, SO₂, CO, l'hétérocycle pouvant être cationique, et/ou substitué par un radical cationique,
- un radical alkylcarbonyle en C₁-C₆, éventuellement substitué,
- un radical -O-CO-R, -CO-O-R, NR-CO-R' ou -CO-NRR' avec R et R' tels que définis précédemment,
- un halogène,
- un radical -NHSO₃H,
- un radical alkyle en C₁-C₄ éventuellement substitué,
- un cycle carboné saturé, insaturé ou aromatique, éventuellement substitué.
- R'₃, R'₄ et R'₅, peuvent former deux à deux un cycle saturé ou non,
• X représente un ion ou groupe d'ions permettant d'assurer l'électronégativité du dérivé de formule (II),
avec la condition qu'au moins un des groupements R'₁ et R'₂ représente un radical cationique.
le procédé de coloration étant mis en oeuvre en l'absence de tout agent oxydant chimique.

2. Procédé selon la revendication 1, **caractérisé par le fait que** les composés de formule (I) sont choisis parmi les composés de sous-formule suivante, et leurs sels d'addition avec un acide ou avec une base : 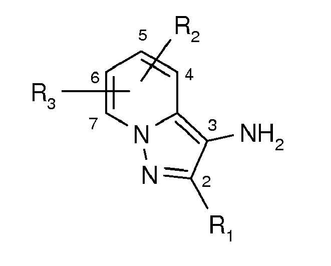 dans laquelle :
R₁, R₂, R₃, identiques ou différents, représentent un atome d'hydrogène ou d'halogène; un radical hydroxyle ; un radical (C₁-C₄)alkyle ; un radical (C₁-C₄)alkylthio ; un radical (C₁-C₄)alkoxy ; un radical -NHSO₃H ; un radical amino ; un radical (C₁-C₄)alkylamino ; un radical di(C₁-C₄)alkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre ; un hétérocycle ; un radical sulfonamide, un radical carbonyle, un radical alcoxy(C₁-C₄)carbonyle, un radical carboxamido, ou un groupement de formule suivante : 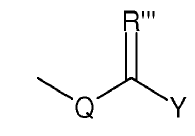 dans lequel R"' représente un atome d'oxygène ou d'azote, X représente un atome d'oxygène, un groupement NH ou NH(C₁-C₄)alkyle, et Y représente un radical hydroxyle, amino, alkyle en C₁-C₄, (C₁-C₄)alkoxy, (C₁-C₄)alkylamino, ou di(C₁-C₄)alkylamino.

3. Procédé selon la revendication 1, **caractérisé par le fait que** les 3-amino pyrazolo-[1,5-a]-pyridines de formule (I) sont choisies parmi :
- la pyrazolo[1,5-a]pyridin-3-ylamine ;
- la 2-acétylamino pyrazolo-[1,5-a] pyridin-3-ylamine ;
- la 2-morpholin-4-yl-pyrazolo[1 ,5-a]pyridin-3-ylamine ;
- l'acide 3-amino-pyrazolo[1 ,5-a]pyridin-2-carboxylique ;
- la 2-méthoxy-pyrazolo[1,5-a]pyridine-3-ylamino ;
- le (3-amino-pyrazolo[1 ,5-a]pyridine-7-yl)-méthanol ;
- le 2-(3-amino-pyrazolo[1 ,5-a]pyridine-5-yl)-éthanol ;
- le 2-(3-amino-pyrazolo[1,5-a]pyridine-7-yl)-éthanol ;
- le (3-amino-pyrazolo[1 ,5-a]pyridine-2-yl)-méthanol ;
- la 3,6-diamino-pyrazolo[1,5-a]pyridine ;
- la 3,4-diamino-pyrazolo[1 ,5-a]pyridine ;
- la pyrazolo[1,5-a]pyridine-3,7-diamine ;
- la 7-morpholin-4-yl-pyrazolo[1 ,5-a]pyridin-3-ylamine ;
- la pyrazolo[1,5-a]pyridine-3,5-diamine ;
- la 5-morpholin-4-yl-pyrazolo[1 ,5-a]pyridin-3-ylamine ;
- le 2-[(3-amino-pyrazolo[1 ,5-a]pyridin-5-yl)-(2-hydroxyéthyl)-amino]-éthanol ;
- le 2-[(3-amino-pyrazolo[1,5-a]pyridin-7-yl)-(2-hydroxyéthyl)-amino]-éthanol ;
- la 3-amino-pyrazolo[1,5-a]pyridine-5-ol ;
- la 3-amino-pyrazolo[1,5-a]pyridine-4-ol ;
- la 3-amino-pyrazolo[1 ,5-a]pyridine-6-ol ;
- la 3-amino-pyrazolo[1,5-a]pyridine-7-ol ;
- la 2-methoxy-6,7-dimethylpyrazolo[1,5-a]pyridin-3-amine ;
- la 2-[(3-aminopyrazolo[1,5-a]pyridin-2-yl)oxy]éthanol ;
- la 4-ethyl-2-methoxy-7-methylpyrazolo[1,5-a]pyridin-3-amine hydrochloride ;
- la 1-(3-aminopyrazolo[1,5-a]pyridin-2-yl)pyrrolidin-3-ol ;
- la 2,2'-[(3-aminopyrazolo[1 ,5-a]pyridin-2-yl)imino]diethanol ;
- la 2-[(3-aminopyrazolo[1,5-a]pyridin-2-yl)amino]ethanol ;
- la N2-(2-pyridin-3-ylethyl)pyrazolo[1 ,5-a]pyridine-2,3-diamine ;
et leurs d'addition avec un acide ou avec une base.

4. Procédé selon la revendication 1 **caractérisé en ce que**, dans la formule (II), Z₁ et/ou Z₂ représente une liaison covalente, un radical -NR'₆(CH₂)_{q}- ou un radical - O(CH₂)ₚ- et R'₁ et/ou R'₂ est un radical cationique.

5. Procédé selon la revendication 4 dans lequel R'₁ ou R'₂ désignent les imidazoles substitués par un radical ammonium quaternaire ou les imidazoliums, les pipérazines substitués par un radical ammonium quaternaire ou les pipéraziniums, les pyrrolidines substitués par un radical ammonium quaternaire ou les pyrrolidiniums, les diazépanes substitués par un radical ammonium quaternaire ou les diazépaniums..

6. Procédé selon la revendication 4 dans lequel R'₁ et R'₂ représentent indépendamment un atome d'hydrogène, ou un groupement trialkylammonium, tri(hydroxyalkyl)ammonium, hydroxyalkyl-dialkyl-ammonium ou di(hydroxyalkyl)alkylammonium.

7. Procédé selon l'une quelconque des revendications précédentes dans lequel les radicaux R'₃, R'₄ et R'₅ de la formule (II) représentent indépendamment un atome d'hydrogène, un radical alkyle en C₁-C₄ pouvant être substitué.

8. Procédé selon l'une quelconque des revendications précédentes dans lequel le composé de formule (II) correspond à 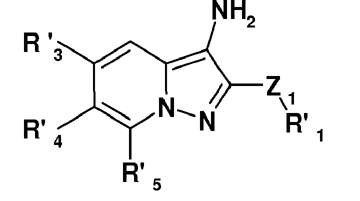 dans laquelle Z₁, R'₁, R'₃, R'₄ et R'₅ sont tels que définis à l'une quelconque des revendications précédentes.

9. Procédé selon la revendication 8 dans laquellelequel Z₁ représente une liaison covalente, un radical -NR'₆(CH₂)_{q}- ou un radical -O(CH₂)ₚ- et R'₁ est un radical cationique.

10. Procédé selon l'une quelconque des revendications précédentes dans lequel le composé de formule (II) est choisi parmi :
| |
|---|
| 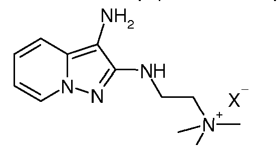 |
| Sel de [2-(3-Amino-pyrazolo[1,5-a]pyridin-2-ylamino)-ethyl]-trimethyl-ammonium |
| 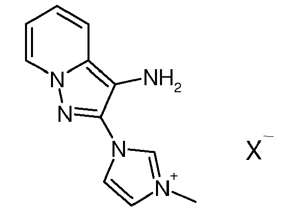 |
| Sel de 3-(3-Amino-pyrazolo[1,5-a]pyridin-2-yl)-1-methyl-3H-imidazol-1-ium |
| 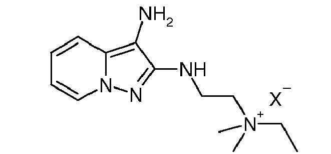 |
| Sel de [2-(3-Amino-pyrazolo[1,5-a]pyridin-2-ylamino)-ethyl]-ethyl-dimethyl-ammonium |
| 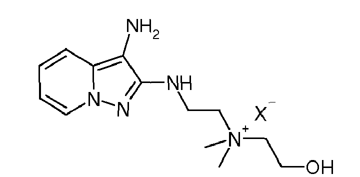 |
| Sel de [2-(3-Amino-pyrazolo[1,5-a]pyridin-2-ylamino)-ethyl]-(2-hydroxy-ethyl)-dimethyl-ammonium |
| 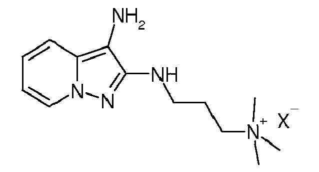 |
| Sel de [3-(3-Amino-pyrazolo[1,5-a]pyridin-2-ylamino)-propyl]-trimethyl-ammonium |
| 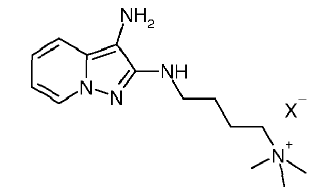 |
| Sel de [4-(3-Amino-pyrazolo[1,5-a]pyridin-2-ylamino)-butyl]-trimethyl-ammonium |
| 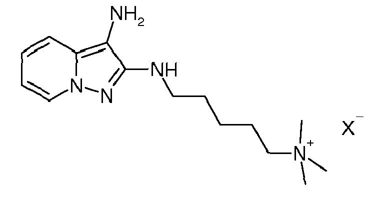 |
| Sel de [5-(3-Amino-pyrazolo[1,5-a]pyridin-2-ylamino)-pentyl]-trimethyl-ammonium |
| 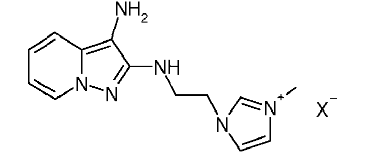 |
| Sel de 3-[2-(3-Amino-pyrazolo[1,5-a]pyridin-2-ylamino)-ethyl]-1-methyl-3H-imidazol-1-ium |
| 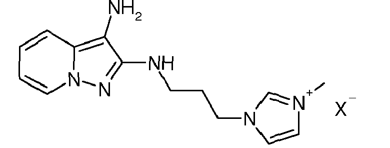 |
| Sel de 3-[3-(3-Amino-pyrazolo[1,5-a]pyridin-2-ylamino)-propyl]-1-methyl-3H-imidazol-1-ium |
| 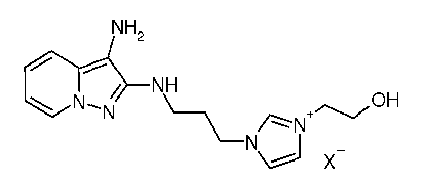 |
| Sel de 3-[3-(3-Amino-pyrazolo[1,5-a]pyridin-2-ylamino)-propyl]-1-(2-hydroxy-ethyl)-3H-imidazol-1-ium |
| 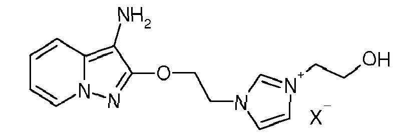 |
| Sel de 3-[2-(3-Amino-pyrazolo[1,5-a]pyridin-2-yloxy)-ethyl]-1-(2-hydroxy-ethyl)-3H-imidazol-1-ium |
| 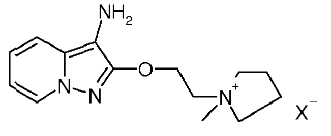 |
| Sel de 1-{2-[(3-aminopyrazolo[1,5-a]pyridin-2-yl)oxy]ethyl}-1-methylpyrrolidinium |
| 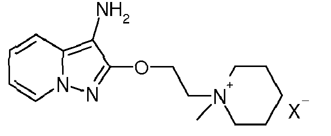 |
| Sel de 1-{2-[(3-aminopyrazolo[1,5-a]pyridin-2-yl)oxy]ethyl}-1-methylpiperidinium |
| 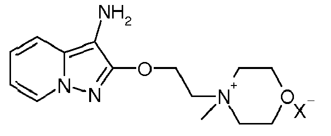 |
| Sel de 4-{2-[(3-aminpyrazolo[1.5-a]pyridin-2-yl)oxy]ethyl}-4-mothylmorpholin-4-ium |
| 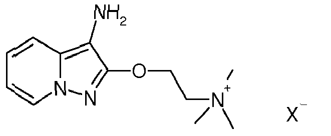 |
| Sel de {2-[(3-aminopyrazolo[1,5-a]pyridin-2-yl)oxy]ethyl}- trimethyl-ammonium |
| 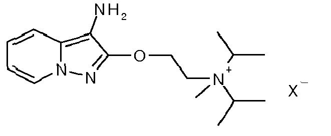 |
| Sel de {2-[(3-aminopyrazolo[1,5-a]pyridin-2-yl)oxy]ethyl}-diisopropyl-methyl-ammonium |
| 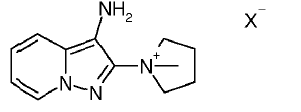 |
| Sel de 1-(3-aminopyrazolo[1,5-a]pyridin-2-yl)-1-methylpyrrolidinium |
| 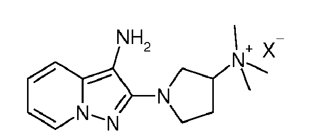 |
| Sel de [1-(3-Amino-pyrazolo[1,5-a]pyridin-2-yl)-pyrrolidin-3-yl]-trimethyl-ammonium |
| 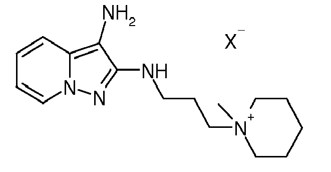 |
| Sel de 1-[3-(3-Amino-pyrazolo[1,5-a]pyridin-2-ylamino)-propyl]-1-methylpiperidinium |
| 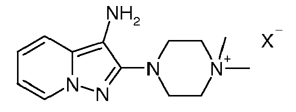 |
| Sel de 4-(3-Amino-pyrazolo[1,5-a]pyridin-2-yl)-1,1-dimethyl-piperazin-1-ium |
| 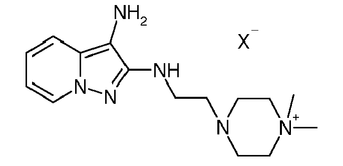 |
| Sel de 4-[2-(3-Amino-pyrazolo[1,5-a]pyridin-2-ylamino)-ethyl]-1,1-dimethyl-piperazin-1-ium |
| 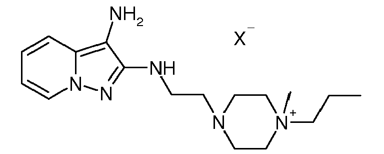 |
| Sel de 4-[2-(3-Amino-pyrazolo[1,5-a]pyridin-2-ylamino)-ethyl]-1-methyl-1-propyl-piperazin-1-ium |
| 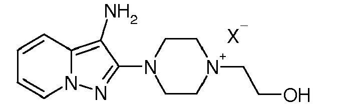 |
| Sel de 4-(3-Amino-pyrazolo[1,5-a]pyridin-2-yl)-1-(2-hydroxy-ethyl)-piperazin-1-ium |
| 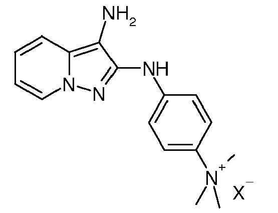 |
| Sel de [4-(3-Amino-pyrazolo[1,5-a]pyridin-2-ylamino)-phenyl]-trimethyl-ammonium |
| 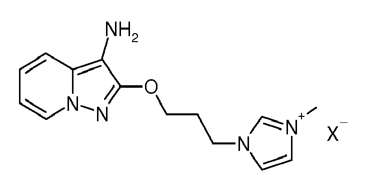 |
| Sel de 3-[3-(3-Amino-pyrazolo[1,5-a]pyridin-2-yloxy)-propyl]-1-methyl-3H-imidazol-1-ium |
| 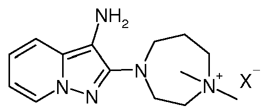 |
| Sel de 4-(3-Amino-pyrazolo[1,5-a]pyridin-2-yl)-1,1-dimethyl-[1,4]diazepan-1-ium |
| 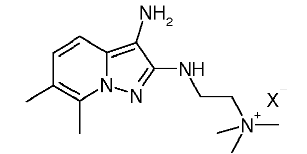 |
| Sel de [2-(3-Amino-6,7-dimethyl-pyrazolo[1,5-a]pyridin-2-ylamino)-ethyl]-trimethyl-ammonium |
| 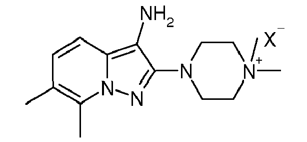 |
| Sel de 4-(3-Amino-6,7-dimethyl-pyrazolo[1,5-a]pyridin-2-yl)-1,1-dimethyl-piperazin-1-ium |
| 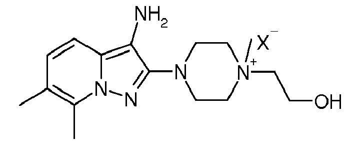 |
| Sel de 4-(3-Amino-6,7-dimethyl-pyrazolo[1,5-a]pyridin-2-yl)-1-(2-hydroxy-ethyl)-1-methyl-piperazin-1-ium |
| 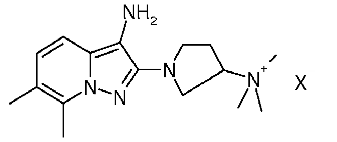 |
| Sel de [1-(3-Amino-6,7-dimethyl-pyrazolo[1,5-a]pyridin-2-yl)-pyrrolidin-3-yl]-trimethyl-ammonium |
| 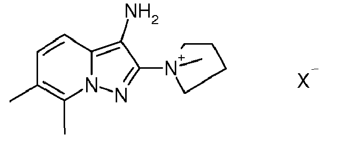 |
| Sel de 1-(3-amino-6,7-dimethylpyrazolo[1,5-a]pyridin-2-yl)-1-methylpyrrolidinium |
| 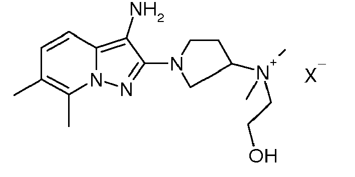 |
| Sel de [1-(3-Amino-6,7-dimethyl-pyrazolo[1,5-a]pyridin-2-yl)-pyrrolidin-3-yl]-(2-hydroxy-ethyl)-dimethyl-ammonium |
| 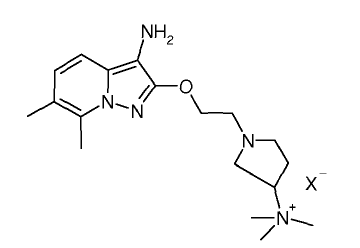 |
| Sel de {1-[2-(3-Amino-6,7-dimethyl-pyrazolo[1,5-a]pyridin-2-yloxy)-ethyl]-pyrrolidin-3-yl}-trimethyl-ammonium |
| 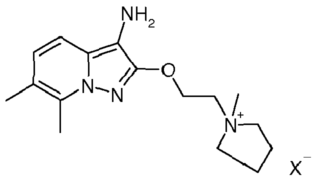 |
| Sel de 1-{2-[(3-amino-6,7-dimethylpyrazolo[1,5-a]pyridin-2-yl)oxy]ethyl}-1-methyl pyrrolidinium |
| 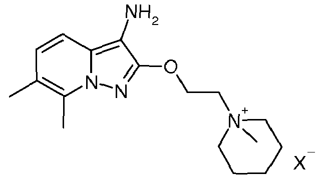 |
| Sel de 1-{2-[(3-amino-6,7-dimethylpyrazolo[1,5-a]pyridin-2-yl)oxy]ethyl}-1-methylpiperidinium |
| 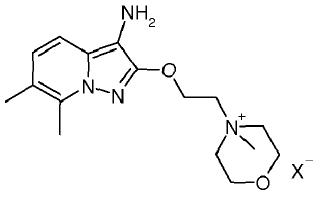 |
| Sel de 4-{2-[(3-amino-6,7-dimethylpyrazolo[1.5-a]pyridin-2-yl)oxy]ethyl}-4-methylmorpholin-4-ium |
| 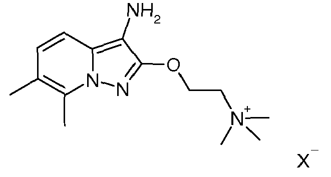 |
| Sel de {2-[(3-amino-6,7-dimethylpvrazolo[1,5-a]pyridin-2-yl)oxy]ethyl}-trimethyl-ammonium |
| 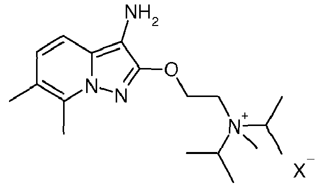 |
| Sel de {2-[(3-amino-6,7-dimethylpyrazolo[1,5-a]pyridin-2-yl)oxy]ethyl}-diisopropyl-methyl-ammonium |
| 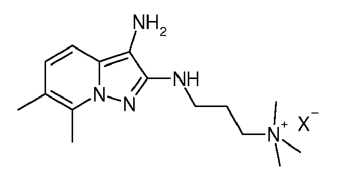 |
| Sel de [3-(3-Amino-6,7-dimethyl-pyrazolo[1,5-a]pyridin-2-ylamino)-propyl]-trimethyl-ammonium |
| 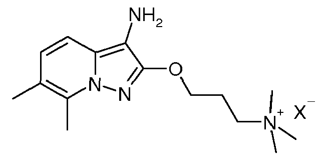 |
| Sel de [3-(3-Amino-6,7-dimethyl-pyrazolo[1,5-a]pyridin-2-yloxy)-propyl]-trimethyl-ammonium |
| 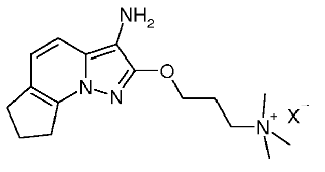 |
| Sel de [3-(3-Amino-7,8-dihydro-6H-cyclopenta[e]pyrazolo[1,5-a]pyridin-2-yloxy)-propyl]-trimethyl-ammonium |
| 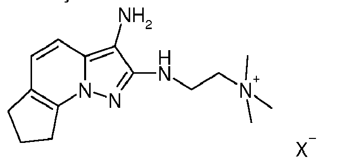 |
| Sel de {2-[(3-amino-7,8-dihydro-6H-cyclopenta[e]pyrazolo[1,5-a]pyridin-2-yl)amino]-ethyl}-trimethyl-ammonium |
| 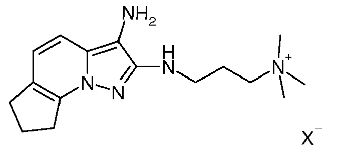 |
| Sel de {3-[(3-amino-7,8-dihydro-6H-cyclopenta[e]pyrazolo[1,5-a]pyridin-2-yl)amino]-propyl}-trimethyl-ammonium |
| 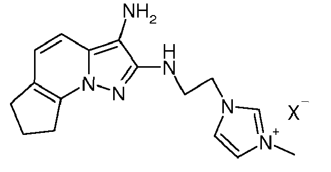 |
| Sel de 1-{2-[(3-amino-7,8-dihydro-6H-cyclopenta[e]pyrazolo[1,5-a]pyridin-2-yl)amino]ethyl}-3-methyl-1H-imidazol-3-ium |
| 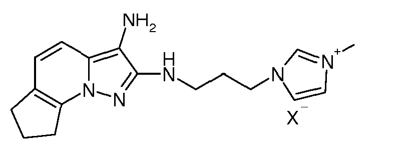 |
| Sel de 1-{3-[(3-amino-7,8-dihydro-6H-cyclopenta[e]pyrazolo[1,5-a]pyridin-2-yl)amino]propyl}-3-methyl-1H-imidazol-3-ium |
| 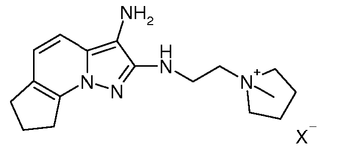 |
| Sel de 1-{2-[(3-amino-7,8-dihydro-6H-cyclopenta[e]pyrazolo[1,5-a]pyridin-2-yl)amino]ethyl}-1-methylpyrrolidinium |
| 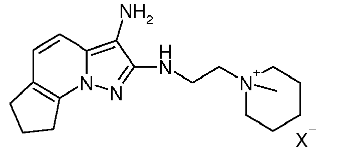 |
| Sel de 1-{2-[(3-amino-7,8-dihydro-6H-cyclopenta[e]pyrazolo[1,5-a]pyridin-2-yl)amino]ethyl}-1-methylpiperidinium |
| 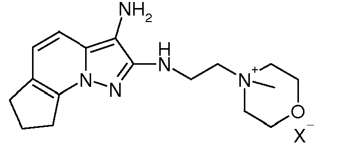 |
| Sel de 4-{2-[(3-amino-7,8-dihydro-6H-cyclopenta[e]pyrazolo[1,5-a]pyridin-2-yl)amino]ethyl}-4-methylmorpholin-4-ium |
| 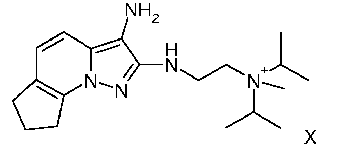 |
| Sel de {2-[(3-amino-7,8-dihydro-6H-cyclopenta[e]pyrazolo[1,5-a]pyridin-2-yl)amino]ethyl}-diisopropyl-methyl-ammonium |
| 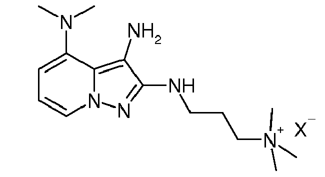 |
| Sel de [3-(3-Amino-4-dimethylamino-pyrazolo[1,5-a]pyridin-2-ylamino)-propyl]-trimethyl-ammonium |
| 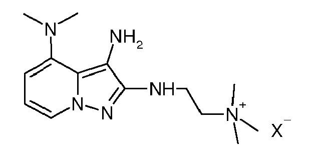 |
| Sel de [2-(3-Amino-4-dimethylamino-pyrazolo[1,5-a]pyridin-2-ylamino)-ethyl]-trimethyl-ammonium |
| 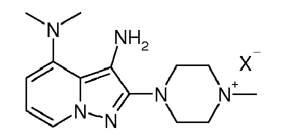 |
| Sel de 4-(3-Amino-4-dimethylamino-pyrazolo[1,5-a]pyridin-2-yl)-1-methyl-piperazin-1-ium |
| 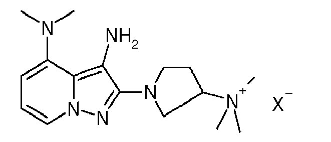 |
| Sel de [1-(3-Amino-4-dimethylamino-pyrazolo[1,5-a]pyridin-2-yl)-pyrrolidin-3-yl]-trimethyl-ammonium |
| 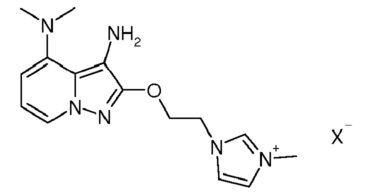 |
| Sel de 3-[2-(3-Amino-4-dimethylamino-pyrazolo[1,5-a]pyridin-2-yloxy)-ethyl]-1-methyl-3H-imidazol-1-ium |
| 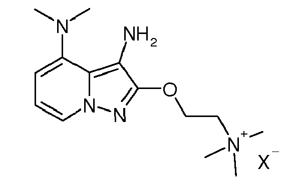 |
| Sel de [2-(3-Amino-4-dimethylamino-pyrazolo[1,5-a]pyridin-2-yloxy)-ethyl]-trimethyl-ammonium |
| 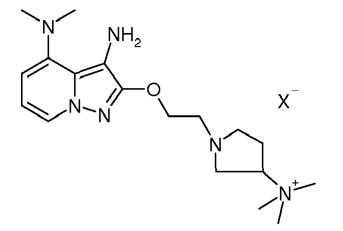 |
| Sel de {1-[2-(3-Amino-4-dimethylamino-pyrazolo[1,5-a]pyridin-2-yloxy)-ethyl]-pyrrolidin-3-yl}-trimethyl-ammonium |
| 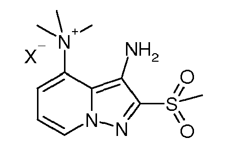 |
| Sel de (3-Amino-2-methanesulfonyl-pyrazolo[1,5-a]pyridin-4-yl)-trimethyl-ammonium |
| 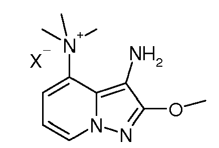 |
| Sel de (3-Amino-2-methoxy-pyrazolo[1,5-a]pyridin-4-yl)-trimethyl-ammonium |

11. Procédé selon l'une quelconque des revendications précédentes comprenant un ou plusieurs coupleurs choisis parmi les métaphénylènediamines, les méta-aminophénols, les métadiphénols, les coupleurs naphtaléniques, les coupleurs hétérocycliques et leurs sels d'addition.

12. procédé selon l'une quelconque des revendications précédentes dans lequel le procédé est mis en oeuvre en présence d'oxygène de l'air et en absence de tout autre agent oxydant chimique.
